## Europäisches Patentamt
## European Patent Office
## Office européen des brevets

(19)

(11) Veröffentlichungsnummer: **0 236 980**
**B1**

(12)    # EUROPÄISCHE PATENTSCHRIFT

(45) Veröffentlichungstag der Patentschrift: **06.06.90**

(51) Int. Cl.⁵: **C 07 C 31/10, C 07 C 29/76**

(21) Anmeldenummer: **87103232.2**

(22) Anmeldetag: **06.03.87**

(54) **Verfahren zum Deodorieren von Isopropylalkohol.**

(30) Priorität: **12.03.86 DE 3608202**

(43) Veröffentlichungstag der Anmeldung:
**16.09.87 Patentblatt 87/38**

(45) Bekanntmachung des Hinweises auf die
Patenterteilung:
**06.06.90 Patentblatt 90/23**

(84) Benannte Vertragsstaaten:
**AT BE CH DE ES FR GB GR IT LI LU NL SE**

(56) Entgegenhaltungen:
**US-A-4 219 685**

(73) Patentinhaber: **RWE-DEA Aktiengesellschaft für**
**Mineraloel und Chemie**
**Überseering 40**
**D-2000 Hamburg 60 (DE)**

(72) Erfinder: **Osterburg, Günther**
**Ahornstrasse 5**
**D-4137 Rheurdt 2 (DE)**
Erfinder: **Gluzek, Karl-Heinz**
**Die Schraag 11**
**D-4234 Alpen 1 (DE)**
Erfinder: **Neier, Wilhelm**
**An der Landwehr 40**
**D-4134 Rheinberg 3 (DE)**

(74) Vertreter: **Schupfner, Gerhard D.**
**Müller, Schupfner & Gauger Karlstrasse 5**
**Postfach 14 27**
**D-2110 Buchholz/Nordheide (DE)**

Courier Press, Leamington Spa, England.

EP 0 236 980 B1

EP 0 236 980 B1

**Beschreibung**

Die Erfindung betrifft ein Verfahren zum Deodorieren von Isopropylalkohol, der durch katalytische Hydratation von Propen hergestellt worden ist, durch Inkontaktbringen mit einem Festbett-Deodorisierungsharz.

Isopropylalkohol wird insbesondere durch Umsetzung von Propen mittels Schwefelsäure oder durch Direkthydratation an sulfonierten Kationenaustauscherharzen hergestellt. Er enthält Spuren von verschiedenen Verbindungen, welche dem technischen Produkt einen unangenehmen Geruch verleihen, der dieses für die industrielle Weiterverarbeitung und insbesondere für den Einsatz in der kosmetischen und pharmazeutischen Industrie ungeeignet macht.

Die als Verursacher des Geruchs anzusehenden Verunreinigungen konnten nur zum Teil durch gaschromatographische Untersuchungen nachgewiesen werden. Ihr Gehalt in dem technischen Alkohol liegt im ppb-Bereich und darunter. Es handelt sich hierbei insbesondere um organische Schwefelverbindungen, wie Schwefelwasserstoff, Carbonylsulfid und Mercaptane. Bisher waren aufwendige Behandlungsverfahren erforderlich, wie Destillations- und Adsorptionsverfahren, um den technischen Alkohol von diesen Substanzen zu befreien. So wurde über viel Jahre eine Deodorierung durch Behandlung mit Aktivkohle vorgenommen, wobei die Aktivkohle in regelmäßigen Zeitabständen durch kostenaufwendige Dampfbehandlung regeneriert werden mußte. Zudem eignen sich nur wenige Kohlen für eine solche Behandlung, da sie entweder zu viel Nebenprodukte, im wesentlichen Aceton, erzeugen, nicht oder nur beschränkt regenierbar oder nicht hinreichend wirksam zur Deodorierung sind.

Aus der US—PS 2 857 436 ist ein Verfahren bekannt, wonach niedere technische Alkohole zur Geruchsverbesserung mit einem feinteiligen Kiesel/Eisen-Material von hoher Oberfläche in Berührung gebracht werden. Nach einer weiteren Ausführungsform des Verfahrens dieser Patentschrift werden die niederen Alkohole nur Geruchsverbesserung mit unglasiertem Porzellan und Stahlwolle in Berührung gebracht.

Gemäß der US—PS 2 729 682 wird versucht, eine Geruchsverbesserung von Isopropylalkohol dadurch herbeizuführen, daß man bei der Herstellung dem Propylenstrom $C_4$- bis $C_6$-Monoolefine hinzugibt, mit Schwefelsäure umsetzt und das Umsetzungsprodukt hydrolisiert und hierbei gleichzeitig das darin enthaltene $C_4$—$C_6$-Olefin zusammen mit den Verunreinigungen, die den Geruch verursachen, bei Temperaturen bis 300°C zu hochsiedenden Verunreinigungen umsetzt. Der gereinigte Isopropylalkohol wird sodann durch extraktive Destillation mit Wasser gewonnen.

Gemäß der deutschen Offenlegungsschrift 28 40 118, die dem US—PS 4 219 685 entspricht, wird eine Deodorierung von $C_2$- und $C_3$-Alkoholen in Gegenwart hydrieraktiver Metalle, insbesondere von Nickel- und Platinmetallen, auf anorganischen Trägermaterialien bei erhöhter Temperatur durchgeführt.

Die Verfahren des Standes der Technik sind entweder umständlich und konstenaufwendig, oder aber es entstehen bei der dort vorgeschlagenen Behandlungsweise weitere Verunreinigungen, insbesondere Ketone und Äther.

Der Erfindung liegt daher die Aufgabe zugrunde, ein Deodorierungsverfahren bereitzustellen, des den unangenehmen Geruch des technischen Produktes weitgehend nimmt, einfach zu handhaben und kostengünstig ist und das nicht seinerseits Nebenprodukte wie insbesondere Ketone und Äther bildet.

Diese Aufgabe wird erfindungsgemäß dadurch gelöst, daß das Deodorierungsharz hergestellt worden ist, indem man

(a) ein starksaures Kationenaustauscherharz mit Palladium in Ionenform belädt und mit einer Mischung von Alkali- oder Erdalkalihydroxid und -halogenid im Verhältnis von Hydroxid zu Halogenid von 95:5 bis 75:25 neutralisiert, wobei die Alkali- oder Erdalkali-Ionen höchstens in äquivalenter Menge zu den noch freien sulfonsauren Gruppen des Kationenaustauscherharzes bereitgestellt werden, oder

(b) ein vollständig mit Alkali- oder Erdalkalihydroxid neutralisiertes starksäures Kationenaustauscherharz mit Palladium in Ionenform belädt und mit Halogenwasserstoffsäure unter Einstellung einer das Harz umgebenden Säurekonzentration von 0,01 n bis 0,5 n nachbehandelt.

Überraschenderweise wurde somit gefunden, daß ein neutralisierend behandeltes, mit Palladium in Ionenform beladenes starksaures Kationenaustauscherharz zur Deodorierung von Isopropylalkohol, der durch katalytische Hydration von Propen hergestellt worden ist, vorzüglich geeignet ist. Die erfindungsgemäße Deodorierung erfolgt durch Überleitung des Alkohols über das Festbett-Deodorierungsharz bei Atmosphärendruck und Umgebungstemperatur. Höhere Drücke können angewendet werden, sind aber nicht erforderlich.

Erhöhte Temperaturen sollten, auch wenn das Deodorierungsharz in praktisch vollständig neutralisierter Form vorliegt, möglichst vermieden werden. Man führt die Deodorierung im allgemeinen bei 0° bis 50°C, insbesondere bei 10 bis 30°C und vorzugsweise bei 15 bis 25°C durch.

Da die Deodorierung sehr schnell verläuft, sind hohe Belastungen des Festbett-Deodorierungsharzes, so von 0,1 bis 15 l Alkohol/h · l Kationenaustauscherharz möglich. Vorzugsweise kann man 0,4 bis 10 l Alkohol/h · l Kationenaustauscherharz durchsetzen.

Die Palladiumbeladung beträgt vorzugsweise 0,1 bis 10 g (berechnet als Metall) je Liter Kationenaustauscherharz und insbesondere 5 bis 8 g (berechnet als Metall) je Liter Kationenaustauscherharz.

Es wird eine vollständige Beladung des starksauren Kationenaustauscherharzes angestrebt, um saure Kontaktstellen weitgehend zu vermeiden. Diese führen zu Nebenreaktionen, und zwar insbesondere zur Ätherbildung und Weiterkondensation von gebildetem Aceton, was unerwünscht ist.

Gemäß einer bevorzugten Ausführungsform des erfindungsgemäßen Verfahrens nach Variante (a) wird das mit Palladium beladene starksaure Kationenaustauscherharz mit einer Mischung von Alkali- oder Erdalkali-hydroxid und -halogenid im Verhältnis von Hydroxid zu Halogenid von 85:15 bis 90:10 neutralisiert. Es wird insbesondere eine Mischung von NaOH/NaCl eingesetzt.

Gemäß einer bevorzugten Ausführungsform des erfindungsgemäßen Verfahrens nach Variante (b) wird die Nachbehandlung mit Halogenwasserstoffsäure unter Einstellung einer Säurekonzentration von 0,01 bis 0,1 n durchgeführt. Es wird insbesondere mit Salzsäure nachbehandelt.

Das erfindungsgemäße Verfahren führt man in besonders wirtschaftlicher Weise durch, indem man Isopropylalkohol einsetzt, der zuvor einer Vordeodorierung an einem mit Silberionen beladenen starksauren Kationenaustauscherharz unterzogen wurde.

Bei den als Trägerharz eingesetzten starksauren Kationenaustauschern handelt es sich um solche auf Kunstharzbasis, insbesondere um Styrol-Divinylbenzol-Mischpolymerisate mit Sulfonsäuregruppen um aromatischen Kern, die auch der Herstellung des Isopropylalkohols aus Propen durch Direkthydratation zum Einsatz kommen. Bevorzugt handelt es sich um Harze vom Typ Amberlyt® 15 der Firma Rohm & Haas. Co., U.S.A. oder Lewatit® SPC 118 der Firma Bayer AG, Leverkusen.

Das verbrauchte Deodorierungsharz kann unter Rückgewinnung des Metalls regeneriert werden.

Zur Geruchsprüfung wird eine Probe in einem reagenzglasähnlichen Glaszylinder mit Schliff und Glasstopfen mit geruchsfreiem Wasser verdünnt, kräftig durchgemischt und der Geruch geprüft. Durch eine dem Geruch entsprechende Benotung von mehreren Personen wird der Alkohol klassifiziert:

| Qualität | | Bewertungsziffer | |
|---|---|---|---|
| IPA SS | = | 1,0—1,4 | |
| IPA S | = | 1,5—2,4 | abnehmende |
| IPA | = | 2,5—3,4 | Qualität |
| IPA K | = | 3,5—4,0 | |

Die endgültige Bewertungsziffer ergibt sich aus der Summe der Punkte, dividiert durch die Anzahl der Prüfer.

Das erfindungsgemäße Verfahren ist sowohl zur Deodorierung des trockenen Isopropylalkohols wie auch zur Deodorierung von Gemischen des Alkohols mit Wasser und insbesondere azeotropen Isopropylalkohols geeignet.

Die folgenden Beispiele erläutern die Erfindung.

Beispiel 1 (Vergleichsbeispiel)

Palladium in der metallischen Form, auf anorganische Träger aufgebracht, kann die in einem technischen Isopropylalkohol enthaltenen schwefelhaltigen Geruchsträger vollständig entfernen. Die katalytische Aktivität des metallischen Palladium führte zu einer unzulässig hohen Umwandlung von Isopropylalkohol (IPA) in Aceton bzw. zu einer entsprechend hohen Verunreinigung von IPA und ist so für die Deodorierung von IPA nicht geeignet.

TABELLE I

| | 1.1 | 1.2 | 1.3 | 1.4 |
|---|---|---|---|---|
| Palladium, Gew.% | 5 | 0,5 | 0,5 | 0,5 |
| Wertigkeit des Metalls | 0 | 0 | 0 | 0 |
| Trägermaterial | $Al_2O_3$ | $Al_2O_3$ | $Al_2O_3$ | $Al_2O_3$ |
| Temperatur, °C | 20 | 20 | 20 | 50 |
| Belastung, 1/V | 0,35 | 0,35 | 1,75 | 1,75 |
| Dauer, Tage | 60 | 1 | 1 | 1 |
| Geruch | 1n | 1 | 1 | 1 |
| UV-Kurve | Keton | Keton | Keton | Keton |
| Keton, ppm | 300 | 300 | 300 | 580 |
| n=neuartiger Fremdgeruch | | | | |

Der Katalysator 1.2—1.4 neigte nach IPA-Behandlung zur Selbstentzündung.

Palladium in der ionischen Form, auf starksaure Kationenaustauscher aufgebracht, zeigte eine ebenso gute Entfernung der schwefelhaltigen Geruchsträger aus IPA wie die metallische Form von Palladium. Nachteilig erwies sich aber auch hier eine durch Temperatur und Zeit begünstigte katalytische

3

Umwandlung von IPA, die insbesondere aus der Kombination von katalytisch aktivem Palladium und den katalytisch wirkenden Säuregruppen des Kationenaustauschers zu unzulässigen Verunreinigungen von IPA durch verschiedene Nebenprodukte führte.

Besipiel 2 (Vergleichsbeispiel)

Ein mit Palladium-Ionen beladener, starksaurer Kationenaustauscher, 7,6 g Pd/l auf Lewatit® SPC 118 der Bayer AG, wurde längere Zeit auf Isopropylalkohol bei Zimmertemperatur einwirken gelassen. Damit wurden zwar die vorher enthaltenen Geruchsträger entfernt, jedoch neben der Ausbildung erheblicher neuer Nebengerüche Diisopropylether und Aceton gebildet und eine UV-spektroskopische nachweisbare Verschlechterung der IPA-Qualität herbeigeführt.

Wurde der gleiche mit Palladium beladene Kationenaustauscher zunächst mit NaOH neutralisiert, bevor die Behandlung von IPA erfolgte, so waren außer eine Bildung von Aceton keine weiteren analytischen Veränderung zu erkennen, siehe Tabelle II.

Beispiel 3 (Vergleichsbeispiel)

Über eine Säule von 150 ml eines mit 0,5 g Pd/l belegten starksauren Kationaustauschers, Lewatit® SPC 118, wurden stündlich bei 50°C 300 ml Isopropylalkohol gegeben. Eine nach 2 h Laufzeit entnommene Alkoholprobe wurde wie folgt beurteilt:

Geruch: Die im technischen IPA enthaltenen Geruchsträger waren vollständig entfernt. Eine neue dumpfe Geruchsnote war festzustellen.

Permanganatzeit: Die Permanganatzeit des IPA hatte sich von 35 Minuten auf 10 Minuten verschlechtert (ASTM D 1363).

Gaschromatographisch wurden 60 ppm Diisopropylether und 120 ppm Aceton nachgewiesen.

UV-spektroskopisch war eine Verschlechterung gegenüber dem Einsatz-IPA zu erkennen.

Wurde der gleiche Versuch mit dem gleichen, aber mit NaOH neutralisierten Deodorierungsharz durchgeführt, so waren außer einer zu hohen Acetonbildung von 220 ppm keine weiteren qualitativ nachteiligen Veränderungen des IPA zu erkennen. Die vorher enthaltenen Geruchsträger waren vollständig entfernt worden.

TABELLE II

Katalytische Nebenwirkungen bei Einsatz von palladium-beladenen Kationenaustauschern (Standversuch)

| Kationenaustauscher (wasserfrei) | | SPC 118[1] | SPC 118[2] |
|---|---|---|---|
| Pd-Beladung | g/l | 7,6 | 7,6 |
| $SO_3^{(-)}$ | | $H^+$ | $Na^+$ |
| Harzmenge | ml | 40 | 40 |
| IPA-Menge | ml | 60 | 60 |
| Standzeit | Tage | 8 | 8 |
| Temperatur | °C | 20 | 20 |
| Beurteilung: GC-Analyse | | | |
| niedere KW | ppm | <10 | <10 |
| IPE | ppm | 30 | <10 |
| Aceton | ppm | 120 | 2600 |

UV-Aufnahme:

[1] Im Bereich von 220—260 mμ zeigten sich erhöhte Extinktionen, die auf unbekannte UV-aktive Nebenprodukte zurückzuführen sind.

[2] Hier zeigte sich lediglich die im Maximum bei 272 mμ bekannte Extinktion für Aceton.

Beispiel 4

Bei der Herstellung von neutralisiertem, mit Palladium beladenen starksauren Kationenaustauscher für die Deodorierung von IPA wurde mit zunehmendem Angebot an Neutralisationsmittel eine zunehmende katalytische Aktivität des Palladiums hervorgerufen.

a) 100 ml eines mit 5 g Palladium/l Harz beladenen, starksauren Kationenaustauschers, Amberlyst® 15, dessen freies Äquivalent an Säuregruppen in einer Stunde zu 100% durch Zugabe von 1 n NaOH bis zum pH-Wert 9,0 im Eluat neutralisiert worden war, wurde zur Deodorierung von Isopropylalkohol eingesetzt. Dazu wurden in einer Säule 7 Tage lang bei 20°C 300 ml IPA/h von oben nach unten über das Deodorierungsharz geleitet. Die Geruchsträger waren vollständig entfernt. Der Acetongehalt betrug konstant 300 ppm.

4

b) 100 ml des oben beschriebenen Harzes, dessen freies Äquivalent an Säuregruppen in einer Stunde zu 85% durch Zugabe von 1 n NaOH bis zum pH-Wert 3,0 im Eluat neutralisiert worden war, wurde zur Deodorierung von IPA eingesetzt. In einer Säule wurden 7 Tage lang bei 20°C 300 ml IPA/h von oben nach unten durchgeleitet. Die Geruchsträger waren vollständig entfernt. Der Acetongehalt betrug konstant 60 ppm.

**Beispiel 5**

Bei der Herstellung von neutralisierten, mit Palladium beladenen, starksauren Kationenaustauscherharzen für die Deodorierung von IPA kann bei Begrenzung des Neutralisationsgrades auf 85% des freien Sulfonsäureäquivalents und unter Zusatz von Chlorid-Ionen zum Neutralisationsmittel eine weitgehende Aktivierung des katalytisch wirksamen Palladiums vermeiden werden.

Als Vergleich wurde ein mit 5,75 g Pd/l Harz beladener stärksaurer Kationenaustauscher, Amberlyst® 15, hergestellt und in mehreren gleichen Teilen mit den in der Tabelle III aufgeführten Neutralisationsmitteln behandelt. Anschließend wurden jeweils 100 ml Harz zur Deodorierung von IPA eingesetzt. Dabei wurden in 48 Stunden bei 20°C 300 ml IPA/h durchgeleitet. Die Geruchsentfernung war bei allen Versuchen gut. Die Acetonbildung zeigt die folgende Tabelle:

TABELLE III

| Neutralisationsmittel | Neutralisations-äquivalent | Aceton ppm |
|---|---|---|
| 1 n NaOH | 85% | 61 |
| 1 n NaOH/NaCl 85:15 | 85% | 5 |
| 1 n NaOH/NaCl 90:10 | 85% | 5 |
| 1 n NaOH/NaCl 95:5 | 85% | 13 |
| 1 n NaOH/NaCl 85:15 | 100% | 50 |

**Beispiel 6**

Die Herstellung neutraler, mit Palladium beladener starksaurer Kationenaustauscher für die Deodorierung von IPA kann auch durch die Beladung von vorneutralisierten oder von neutralen Lieferformen kommerzieller Kationenaustauscher erfolgen.

Auch hierbei ist eine katalytische Aktivierung von Palladium nicht auszuschließen. Durch Nachbehandlung mit verdünnter Salzsäure (0,1 n) bis pH-Wert 2 im Eluat wird das katalytisch wirksame Palladium als Hexachloropalladinat abgestoßen und die gewünschte katalytische Inaktivität des Deodorierungsharzes erreicht. 85—90% des genannten Sulfonsäureäquivalents liegen nach dieser Behandlung in der gebundenen neutralen Form vor. 600 l eines so hergestellten, neutralisierten, mit 5 g Pd/l Harz beladenen starksauren Kationenaustauschers vom Typ Amberlyst® 15 wurden zur Deodorierung von IPA eingesetzt. In 142 Tagen wurden 10 224 m³ IPA mit einer Belastung von 3 m³IPA/h bei einer Mitteltemperatur von 20°C von oben nach unten durch das Harzbett geleitet. Die im technischen IPA enthaltenen Geruchsträger wurden vollständig entfernt. Eine Acetonbildung wurde nicht beobachtet.

**Beispiel 7**

In zwei in Serie geschaltete Türme von 0,2 m Durchmesser und 1,0 m Höhe wurden im ersten Turm 30 l eines neutralen, mit 8 g Silber/l Harz beladenen, starksauren Kationenaustauschers vom Typ Lewatit® SPC 118 und im zweiten Turm 30 l eines mit 7,6 g Pd/l Harz beladenen starksauren Kationenaustauschers vom Typ Amberlyst® 15, dessen freies Äquivalent an Säuregruppen zu 90% neutralisiert war, eingesetzt.

Über beide Türme wurde mit einem Durchsatz von 200 l/h ein durch katalytische Hydratation von Propen hergestellter, durch Destillation gereinigter und getrockneter, geruchlich aber unzureichender Isopropylalkohol geleitet.

Dieser Durchsatz entspricht in jedem Turm einer Belastung von 6,6 l/l Harz. Sowohl nach dem ersten Turm mit silberbeladenem Harz als auch nach dem zweiten Turm mit palladiumbeladenem Harz konnten keine analytisch erfaßbaren Veränderungen in dem IPA nachgewiesen werden. Die olfaktorische Beurteilung bis zu einer Laufzeit von 56 Tagen ergab, daß die im Einsatz-IPA enthaltenen Geruchsträger nach dem ersten Turm weitgehend (Geruchsnote 1,5—2,0), nach dem zweiten Turm jedoch vollständig (Geruchsnote 1,0) entfernt waren.

Die beiden Deodorierungsharze zeigten auch nach 56 Tagen noch kein Nachlassen ihrer Deodorierungseigenschaften.

**Patentansprüche**

1. Verfahrem zum Deodorieren von Isopropylalkohol, der durch katalytische Hydratation von Propen hergestellt worden ist, durch Inkontaktbringen mit einem Festbett-Deodorierungsharz dadurch gekennzeichnet, daß das Deodorierungsharz hergestellt worden ist, indem man

(a) ein starksaures Kationenaustauscherharz mit Palladium in Ionenform baladt und mit einer Mischung von Alkali- oder Erdalkalihydroxid und -halogenid im Verhältnis von Hydroxid zu Halogenid von 95:5 bis 75:25 neutralisiert, wobei die Alkali- oder Erdalkali-Ionen höchstens in äquivalenter Menge zu den noch freien sulfonsauren Gruppen des Kationenaustauscherharzes bereitgestellt werden, oder

(b) ein vollständig mit Alkali- oder Erdalkalihydroxid neutralisiertes starksaures Kationenaustauscherharz mit Palladium in Ionenform baladt und mit Halogenwasserstoffsäure unter Einstellung einer das Harz umgebenden Säurekonzentration von 0,01 n bis 0,5 n nachbehandelt.

2. Verfahren nach Anspruch 1, dadurch gekennzeichnet, daß man das mit Palladium beladene starksaure Kationenaustauscherharz mit einer Mischung von Alkali- oder Erdalkali-hydroxid und -halogenid im Verhältnis von Hydroxid zu Halogenid von 85:15 bis 90:10 neutralisiert.

3. Verfahren nach Anspruch 1 oder Anspruch 2, dadurch gekennzeichnet, daß man mit einer Mischung von NaOH/NaCL neutralisiert.

4. Verfahren nach Anspruch 1, dadurch gekennzeichnet, daß man mit Halogenwasserstoffsäure unter Einstellung einer Säurekonzentration von 0,01 bis 0,1 nachbehandelt.

5. Verfahren nach einem der vorhergehenden Ansprüche, dadurch gekennzeichnet, daß man mit Salzsäure nachbehandelt.

6. Verfahren nach einem der vorhergehenden Ansprüche, dadurch gekennzeichnet, daß man das Kationenaustauscherharz mit Palladiumionen in einer Menge von 0,1 bis 10 g, vorzugsweise 5 bis 8 g, (berechnet als Metall) je Liter Harz belädt.

7. Verfahren nach einem der vorhergehenden Ansprüche, dadurch gekennzeichnet, daß man 0,4 bis 10 Liter Alkohol/Liter Harz und Stunde in Kontakt bringt.

8. Verfahren nach einem der vorhergehenden Ansprüche, dadurch gekennzeichnet, daß man die Deodorierung bei 10 bis 30°C und vorzugsweise bei 15 bis 25°C durchführt.

9. Verfahren nach einem der vorhergehenden Ansprüche, dadurch gekennzeichnet, daß man Isopropylalkohol einsetzt, der zuvor einer Vordeodorierung an einem mit Silberionen beladenen starksauren Kationenaustauscherharz unterzogen worden ist.

**Revendications**

1. Procédé de désodorisation d'alcool isopropylique produit par l'hydratation catalytique de propène, en mettant l'alcool en contact avec une résine désodorisante disposée sous forme de lit fixe, caractérisé en ce qu'on met en oeuvre la résine désodorisante de la façon suivante:

(a) une résine échangeuse de cations fortement acide est chargée de palladium sous forme d'ions et est neutralisée avec un mélange d'hydroxyde et d'halogénure alcalin ou alcalino-terreux, le rapport hydroxyde/halogénure se situant entre 95:5 et 75:25, en mettant à disposition au maximum une quantité d'ions alcalins ou alcalino-terreux équivalente à la quantité de groupes acidosulfoniques encore libres de l'échangeur de cations, ou

(b) une résine échangeuse de cations fortement acide, neutralisée en totalité avec de l'hydroxyde alcalin ou alcalino-terreux, est chargée de palladium sous forme d'ions et est traitée ultérieurement par l'haloacide, en maintenant le milieu acide entre 0,01 n et 0,5 n.

2. Procédé selon la revendication 1, caractérisé en ce qu'on neutralise l'échangeur de cations palladié fortement acide avec un mélange d'hydroxyde et d'halogénure alcalin ou alcalino-terreux, le rapport hydroxyde/halogénure se situant entre 85:15 et 90:10.

3. Procédé selon la revendication 1 ou 2, caractérisé en ce qu'on réalise la neutralisation avec un mélange de NaOH/NaCl.

4. Procédé selon la revendication 1, caractérisé en ce qu'on utilise de l'haloacide pour le traitement ultérieur, la concentration de l'acide étant maintenue entre 0,01 et 0,1 n.

5. Procédé selon l'une des revendications précédentes, caractérisé en ce qu'on utilise de l'acide hydrochlorique pour le traitement ultérieur.

6. Procédé selon l'une des revendications précédentes, caractérisé en ce qu'on charge l'échangeur de cations de 0,1 à 10 g d'ions de palladium, de préférence de 5 à 8 g (exprimé en métal), par litre de résine.

7. Procédé selon l'une des revendications précédentes, caractérisé en ce qu'on met en contact 0,4 à 10 litres d'alcool/litre de résine et heure.

8. Procédé selon l'une des revendications précédentes, caractérisé en ce qu'on réalise la désodorisation à 10—30°C, de préférence à 15—25°C.

9. Procédé selon l'une des revendications précédentes, caractérisé en ce qu'on utilise de l'alcool isopropylique qui a été désodorisé préalablement en utilisant un échangeur de cations fortement acide chargé d'ions d'argent.

**Claims**

1. A process for deodorizing isopropyl alcohol produced by the catalytic hydration of propene which comprises contacting said isopropyl alcohol with a fixed-bed deodorizing resin wherein the deodorizing resin has been produced by

(a) loading a strongly acidic cation exchange resin with palladium in ionic form, neutralizing with a mixture of alkali- or alkaline earth hydroxide and -halide at a hydroxide/halide ratio of 95:5 to 75:25, the alkali- or alkaline earth ions being made available at most at the equivalent amount relative to the yet free sulfonic acid groups of the cation exchange resin, or by

(b) loading a strongly acidic cation exchange resin completely neutralized with alkali- or alkaline earth hydroxide with palladium in ionic form and subjecting it to aftertreatment with halogen hydracid while adjusting the acid concentration surrounding the resin at 0.01 n to 0.5 n.

2. A process according to Claim 1 which comprises neutralizing the palladium-loaded strongly acid cation exchange resin with a mixture of alkali- or alkaline earth hydroxide and -halide at a hydroxide/halide ratio of 85:15 to 90:10.

3. A process according to Claim 1 or 2 wherein a mixture of NaOH/NaCl is employed for neutralization.

4. A process according to Claim 1 in which said aftertreatment with halogen hydracid is conducted at an acid concentration ranging from 0.01 to 0.1 n.

5. A process according to any one of the preceding claims in which said aftertreatment is conducted with hydrochloric acid.

6. A process according to any one of the preceding claims in which said cation exchange resin is loaded with palladium ions in an amount ranging from 0.1 to 10 g, preferably 5 to 8 g (calculated as metal) per liter of resin.

7. A process according to any one of the preceding claims in which the treatment rate ranges from about 0.4 to 10 l of alcohol/liter of resin · h.

8. A process according to any one of the preceding claims in which said deodorization is conducted at 10 to 30°C, preferably 15 to 25°C.

9. A process according to any one of the preceding claims in which said isopropyl alcohol is first subjected to predeodorization on a silver ions-loaded strongly acidic cation exchange resin.